# EUROPÄISCHE PATENTANMELDUNG

(11) **EP 1 223 168 A1**
(43) Veröffentlichungstag der Anmeldung: **17.07.2002**
(21) Anmeldenummer: 01127611.0
(22) Anmeldetag: 20.11.2001
(51) Int. Cl.: C07D 307/66, C07D 307/60, C07B 37/10, C07B 53/00

(54) **Verfahren zur Herstellung von enantiomerenangereicherten Amino- und Hydroxyfuranonen**

(30) Priorität: 16.01.2001 AT 612001
(71) Anmelder: DSM Fine Chemicals Austria Nfg GmbH & Co KG, 4021 Linz (AT)
(72) Erfinder: Pöchlauer, Peter, 4040 Linz (AT); Riebel, Peter, 94099 Ruhstorf a.d.Rott (DE); Mayrhofer, Herbert, 4210 Engerwitzdorf (AT); Wirth, Irma, 4470 Enns (AT)
(74) Vertreter: Klostermann, Ingrid

(57) **Zusammenfassung**

Verfahren zur Herstellung von enantiomeren-angereicherten Aminofuranonen und Hydroxyfuranonen, bei welchem ein enantiomeren-angereichertes Cyanhydrin mittels eines Acylierungsmittels acyliert, anschließend bei 40 bis 90°C in Anwesenheit von Zink oder einer Zinkverbindung zu dem korrespondierenden enantiomeren-angereicherten Aminofuranon cyclisiert wird, welches gegebenenfalls durch saure Hydrolyse in das entsprechende enantiomeren-angereicherte Hydroxyfuranon überführt wird.

## Beschreibung

Amino- und Hydroxyfuranone stellen aufgrund ihrer biologischen Aktivitäten wichtige Ausgangsprodukte bei der Herstellung von Verbindungen mit antibiotischer, blutdrucksenkender oder tumorhemmender Wirkung , sowie von Fungiziden, Insectiziden u.s.w. dar.
Die meisten Amino- und Hydroxyfuranone, die ein chirales Zentrum aufweisen, werden jedoch nur in der Form ihrer Racemate eingesetzt jedoch zeigt üblicherweise nur ein Enantiomer in einer racemischen Mischung einer biologisch aktiven Verbindung die gewünschte Wirkung.
Einige Synthesen für optisch aktive Hydroxyfuranone (Tetronsäuren), ausgehend von enantiomerenreinen Cyanhydrinen durch Anwendung der Blaise Reaktion, wurden bereits beschrieben, wobei eine partielle Racemisierung bei der Synthese von racemisierungsanfälligen Verbindungen, wie etwa 5-Aryl-4-hydroxytetronsäuren beobachtet wird (Effenberger, Tetrahedron Asymmetry 9 (1998) 817-825).
Optisch aktive Tetronsäuren werden bisher meistens durch Dieckmann Cyclisierung, ausgehend von enantiomerenreinen Hydroxysäuren, synthetisiert (Effenberger, Tetrahedron Asymmetry 9 (1998) 817-825).
Andere Methoden, ausgehend von Hydroxysäureestern, erfordern tiefe Reaktionstemperaturen von bis zu -78 °C (Witiak, J. Org. Chem. 1990, 55, 1112-1114).
Von Momose, Heterocycles, Vol. 51, 6,1999 wird die Synthese von optisch aktiven 4-Hydroxy-2(5H)-furanon-Derivaten (Tetronsäuren) durch Reaktion von 2-Acyloxyestern mit Zink unter Bildung der Reformatskyverbindung und intramolekularem Ringschluß beschrieben. Dabei kommt es zu einer Nebenreaktion, wobei entstehender Alkohol (beim Ringschluß) die Reformatskyverbindung zerstört und in der Folge die Ausbeuten nur maximal 50 % betragen (Brandänge, ACTA Chem. Scand., 1995, 49,922-928).

Literaturbekannt ist auch, dass Tetronsäuren durch saure Hydrolyse aus 4-Aminofuranonen gewonnen werden können (C. Veronese, Heterocycles, Vol. 32, No 11,1991; Nishide, Tetrahedron Vol. 50, No. 28, 8337-8346, 1994).

Die Darstellung von racemischen 4-Amino-2(5H)-furanon (Aminofuranonen), Derivaten von Cyanhydrinen ist durch intermolekulare Reaktion mit Magnesiumenolaten beschrieben. Weiters werden Cyclisierungsreaktionen an Acyloxynitrilen mit Lithiumamiden bei -78 °C oder mit NaH in THF bei Rückflußtemperatur durchgeführt. Diese Reaktionen sind jedoch nur auf Aldehydcyanhydrine anwendbar (Hiyama, Bull. Chem. Soc. Jpn., 60, 2139-2150, 1987) und nur bei Anwendung der tiefen Reaktionstemperatur können enantiomerenreine Cyanhydrine ohne Racemisierung umgesetzt werden (Ohta, Tetrahedron Letters, Vol. 29, No. 52, pp 6957-6960,1988)
Der Verlust von Enantiomerenreinheit bei Cyclisierungen unter Verwendung von NaH wird beispielsweise in T. Ross Kelly, Tetrahedorn Letters, Vol. 26,18, 2173-2176, 1985 beschrieben.

Die Cyclisierung von Acyloxynitrilen mit Zink bei etwa 60 °C ist für racemische Cyanhydrine in Ausbeuten von ca. 90 % in Chem. Abstr. 109: 110244 (JP 63093774) beschrieben.

Aufgabe der vorliegenden Erfindung war es, ein Verfahren zu finden, das optisch aktive, enantiomeren-angereicherten Aminofuranone und in Folge optisch aktive, entantiomeren-angereicherten Hydroxyfuranone in hohen Ausbeuten und ohne Racemisierung liefert.

Unerwarteterweise konnte ein Verfahren gefunden werden, durch das auch enantiomeren-angereicherte Cyanhydrine nach Acylierung durch intramolekulare Reformatskyreaktion mit Zink bei 60 °C ohne Racemisierung zu optisch aktiven Aminofuranonen, die als solches schon wichtige Verbindungen sind (Hiyama, Tetrahedron Letters, Vol. 26, No. 20, 2459-2462, 1985), in guten Ausbeuten umgesetzt werden.

Die erhaltenen Aminofuranone können weiters in optisch aktive Hydroxyfuranone überführt werden.

Gegenstand der vorliegenden Erfindung ist demnach ein Verfahren zur Herstellung von enantiomeren-angereicherten Aminofuranonen und Hydroxyfuranonen, das dadurch gekennzeichnet ist, dass
a) ein optisch aktives Cyanhydrin mittels eines Acylierungsmittels acyliert, anschlieβend
b) bei 40 bis 90°C in Anwesenheit von Zink oder einer Zinkverbindung zu dem korrespondierenden enantiomeren-angereicherten Aminofuranon cyclisiert wird, welches
c) gegebenenfalls durch saure Hydrolyse in das entsprechende enantiomerenangereicherte Hydroxyfuranon überführt wird.

In Stufe a) werden optisch aktive Cyanhydrine acyliert.
Geeignete Ausgangsverbindungen sind dabei optisch aktive Cyanhydrine, die beispielsweise durch Reaktion eines Aldehydes oder eines Ketones, eines Cyanidgruppendonors und einer Hydroxynitrillyase erhalten werden.

Unter Aldehyden sind dabei aliphatische, aromatische oder heteroaromatische Aldehyde zu verstehen. Aliphatische Aldehyde sind dabei gesättigte oder ungesättigte aliphatische, geradkettige, verzweigte oder cyclische Aldehyde. Bevorzugte aliphatische Aldehyde sind geradkettige Aldehyde mit insbesondere 2 bis 18 C-Atomen, bevorzugt von 2 bis 12, die gesättigt oder ein- oder mehrfach ungesättigt sind. Der Aldehyd kann dabei sowohl C-C-Doppelbindungen als auch C-C-Dreifachbindungen aufweisen. Der Aldehyd kann unsubstituiert oder durch unter den Reaktionsbedingungen inerte Gruppen, beispielsweise durch gegebenenfalls substituierte Aryl- oder Heteroarylgruppen wie Phenyl - oder Indolylgruppen, durch Halogen-, Ether-, Alkohol-, Acyl-, Carbonsäure-, Carbonsäureester-, Nitro- oder Azidogruppen substituiert sein.
Beispiele für aromatische oder heteroaromatische Aldehyde sind Benzaldehyd bzw. verschieden substituierte Benzaldehyde wie etwa 3,4-Difluorbenzaldehyd, 3-Phenoxybenzaldehyd, 4-Fluor-3-phenoxybenzaldehyd, weiters Furfural, Anthracen-9-carbaldehyd, Furan-3-carbaldehyd, Indol-3-carbaldehyd, Naphthalin-1-carbaldehyd, Phthaldialdehyd, Pyrazol-3-carbaldehyd, Pyrrol-2-carbaldehyd, Thiophen-2-carbaldehyd, Isophthalaldehyd u.s.w..
Ketone sind aliphatische, aromatische oder heteroaromatische Ketone, bei denen das Carbonylkohlenstoffatom ungleich substituiert ist. Unter aliphatischen Ketonen sind gesättigte oder ungesättigte, geradkettige, verzweigte oder cyclische Ketone zu verstehen. Die Ketone können gesättigt oder ein- oder mehrfach ungesättigt sein. Sie können unsubstituiert, oder durch unter Reaktionsbedingungen inerte Gruppen, beispielsweise durch gegebenenfalls substituierte Aryl- oder Heteroarylgruppen wie Phenyl- oder Indolylgruppen, durch Halogen-, Ether-, Alkohol-, Acyl-, Carbonsäure-, Carbonsäureester-, Nitro- oder Azidogruppen substituiert sein.
Beispiele für aromatische oder heteroaromatische Ketone sind Acetophenon, Indolylaceton u.s.w..
Die eingesetzten optische aktiven Cyanhydrine enthalten die (S)- oder (R)-Form in einem Anteil von größer 95% und lassen sich durch Formel (I) wie folgt darstellen:

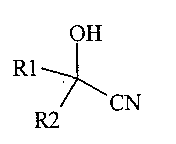

R1 und R2 leiten sich von den oben angeführten Aldehyden und Ketonen ab und bedeuten somit bevorzugt H, wobei nur einer der beiden Reste R1 oder R2 H bedeuten kann; sowie lineares, verzweigtes oder cyclisches C₂-C₁₈-Alkyl, C₂-C₁₈-Alkylen oder C₂-C₁₈-Alkyliden, die gegebenenfalls durch Halogen-, Ether-, Alkohol-, Acyl-, Carbonsäure-, Carbonsäureester-, Nitro- oder Azidogruppen oder durch gegebenenfalls substituierte Aryl- oder Heteroarylgruppen ein- oder mehrfach substituiert sein können oder gegebenenfalls substituiertes Aralkyl, Aryl oder Heteroaryl.

Besonders bevorzugt sind optisch aktive aliphatische und aromatische, Cyanhydrine, wie etwa (R)- oder (S)-3-Phenoxybenzaldehydcyanhydrin, (R)- oder (S)-4-Fluor-3-phenoxybenzaldehyd-cyanhydrin, (R)- oder (S)-3,4-Difluorbenzaldehyd-cyanhydrin, (R)- oder (S)-2-Hydroxy-2,3-dimethylbutannitril, (R)- oder (S)-2-Hydroxy-2-methylpentannitril, (R)- oder (S)-2-Hydroxynonannitril, (R)- oder (S)-2-Hydroxy-2-methylphenylacetonitril, (R)- oder (S)-Mandelsäurenitril.

Die Acylierung erfolgt unter üblichen, aus dem Stand der Technik, beispielsweise aus Takefumi Momose, Heterocycles, Vol. 51, No. 6, 1999 bekannten Bedingungen.
Die Reaktionstemperatur liegt demnach bevorzugt zwischen 20 und 50°C. Das molare Verhältnis von Cyanhydrin zu Acylierungsmittel liegt bevorzugt zwischen 1:1 und 1:3. Als Lösungsmittel eignen sich beispielsweise gegebenenfalls halogenierte Kohlenwasserstoffe, wie etwa Cyclohexan, Xylol, Toluol, Chloroform, Dichlormethan, Chlorbenzol, Ether, Ester, Pyridin u.s.w. oder Gemische davon. Die Acylierung erfolgt bevorzugt unter Basenkatalyse mit Pyridin oder Triethylamin u.s.w. mittels eines Halogencarbonsäurehalogenids der Formel lla 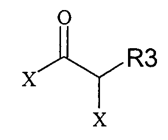 oder Halogencarbonsäureanhydrids der Formel IIb 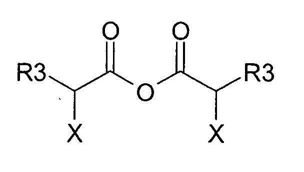 wobei R3 einen aliphatischen, linearen oder verzweigten C₁ bis C₁₈-Alkylrest oder einen Arylrest mit 6 bis 20 C-Atomen und X ein Halogenid aus der Gruppe Fluor, Brom, Chlor und Jod bedeutet,
wodurch Verbindungen der Formel (III) 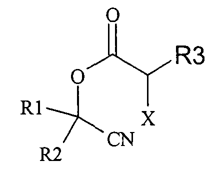 erhalten werden, in der R1, R2, R3 und X wie oben definiert sind.

In Schritt b) erfolgt im Anschluss an die Acylierung, die Cyclisierung der Verbindung der Formel III in Gegenwart von Zink, welches beispielsweise mit Jod oder Kupfer aktiviert werden kann. Bevorzugt wird nur Zn verwendet. Die Cyclisierungstemperaturen liegen bei 40 - 90°C, bevorzugt bei 50 bis 75°C. Zn wird dabei in einer Menge von 1-3 Äquivalenten, bevorzugt von 1 bis 1,5 Äquivalente eingesetzt. Nach einer Reaktionszeit von 0,5 - 3 Stunden werden die korrespondierenden Aminofuranone der Formel (IV) 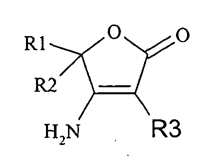 in der R1, R2 und R3 wie oben definiert sind, erhalten.
Als Lösungsmittel eignen sich aliphatische oder aromatische Kohlenwasserstoffe, wie etwa Cyclohexan, Xylol, Toluol, Benzol, Ether, wie etwa Diethylether, Methyl-tert. Butylether, Diisopropylether, Dibutylether, Tetrahydrofuran, u.s.w. oder Gemische davon.

Die Aminofuranone können sodann aus dem Reaktionsgemisch isoliert werden, beispielsweise durch Extraktion, Abdestillieren des Lösungsmittels u.s.w., wodurch die gewünschten Aminofuranone in Ausbeuten bis zu über 90% und in einer Enantiomerenreinheit von >90 % erhalten werden, ohne dass eine Racemisierungsreaktion aufgetreten ist.

Die Aminofuranone können gewünschtenfalls in die entsprechenden optisch aktiven Hydroxyfuranone überführt werden (Schritt c), wobei dies nach Isolierung des jeweiligen Aminofuranons oder jedoch auch ohne Isolierung des Aminofuranons aus dem Reaktionsgemisch, sofort im Anschluss an Schritt b) erfolgen kann.

Schritt c), die saure Hydrolyse, wird analog dem Stand der Technik durchgeführt, und erfolgt beispielsweise in THF/HCI oder mittels basischer Hydrolyse. Bevorzugt wird die Hydrolyse in THF/HCI, besonders bevorzugt mit 15-20 % iger Salzsäure, durchgeführt.
Die entsprechenden Hydroxyfuranone werden sodann aus dem Reaktionsgemisch auf übliche Weise isoliert, beispielsweise durch Extraktion, Abdestillieren des Lösungsmittels u.s.w., und in hohen Ausbeuten bis zu 75 % , sowie in einer Enantiomerenreinheit von > 90 % erhalten.

Das erfindungsgemäße Verfahren zeichnet sich besonders vorteilhaft gegenüber anderen, aus dem Stand der Technik bekannten Cyclisierungsreaktionen aus, da die Cyclisierung durch intramolekularer Reformatskyreaktion mit Zink bzw. Zinkverbindungen sowohl für Aldehydcyanhydrine als auch bei Ketoncyanhydrine angewendet werden kann, sowie keine extremen Reaktionstemperaturen erforderlich sind und auch bei 5-Aryl-4-hydroxytetronsäuren keine Racemisierung beobachtet wird.

### Beispiel 1:Acylierung von Cyanhydrinen:

6,0 g (45,1 mmol) (S)-2-Hydroxyphenylacetonitril mit 98,8 %ee wurden in 25 ml Dichlormethan gelöst und auf 1 °C abgekühlt. Anschließend wurden 11,7 g (54,1 mmol) 2-Brompropionsäurebromid innerhalb von 10 Minuten zugetropft. Nach Zugabe von 0,27 g (2,2 mmol) Dimethylaminopyridin wurden 5,5 g (54,1 mmol) Triethylamin bei einer Temperatur von unter 5 °C zugetropft. Nach einer Rührzeit von 1,75 Stunden bei 5 °C wurde auf Raumtemperatur erwärmt und noch 22 Stunden lang gerührt.
Anschließend wurde mit 55 ml Wasser extrahiert und die Phasen getrennt. Weiters wurde die organische Phase zweimal mit 30 ml gesättigter Natriumhydrogencarbonatlösung und einmal mit gesättigter Natriumchloridlösung extrahiert. Abschließend wurde die organische Phase mit Natriumsulfat getrocknet und das Lösungsmittel vollständig abdestilliert.
Als Rückstand blieben 10,3 g (85,2 % Ausbeute) (S)-2-(2-Brompropionyloxy)-phenylacetonitril.

### Beispiel 2: Cyclisierung optisch aktiver Cyanhydrine zu den Aminofuranonen:

Zur Aktivierung wurde das verwendete Zink mit 10 %iger Salzsäure, Aceton und Diethylether nacheinander gewaschen und durch Rühren unter reduziertem Druck getrocknet.
Es wurden 0,40 g (6,12 mmol) aktiviertes Zink unter Argon in den Reaktionskolben eingewogen und mit 2 ml Tetrahydrofuran versetzt. Danach wurde unter Inertisierung mit Argon auf ca. 65 °C erwärmt. Innerhalb von 15 min wurde eine Lösung von 5,56 mmol des entsprechenden optisch aktiven Acyloxynitrils (siehe Tabelle 1) in 1 ml THF zugetropft. Nach einer Rührzeit von 2 Stunden bei ca. 65 °C wurde abgekühlt und bei einer Temperatur von unter 0 °C 2 ml gesättigte Ammonchloridlösung zugetropft. Anschließend wurde auf Raumtemperatur erwärmt, mit Ethylacetat versetzt und die Phasen getrennt. Die wäßrige Phase wurde noch einmal mit Ethylacetat extrahiert. Die organischen Phasen wurden getrocknet und vom Lösungsmittel befreit. Die Enantiomerenanalytik erfolgte mittels GC an einer Cyclodextrinsäule.

**Tabelle 1:**

| Acyloxynitril | ee | Aminofuranon | ee |
|---|---|---|---|
| (S)-2-(2-Brompropionyloxy)-phenylacetonitril | > 98 % | (5S)-4-Amino-3-methyl-5-phenyl-2(5H)-furanon | 96 % |
| | | | |
| (S)-2-(2-Brompropionyloxy)-nonanonitril | 93 % | (5S)-4-Amino-3-methyl-5-heptyl-2(5H)-furanon | 93 % |
| | | | |
| (S)-2-(2-Brompropionyloxy)-2-phenylpropionitril | 96 % | (55)-4-Amino-3,5-dimethyl-5-phenyl-2(5H)-furanon | 96 % |

### Beispiel 3: Cyclisierung optisch aktiver Cyanhydrine zu den Hydroxyfuranonen:

Zur Aktivierung wurde das verwendete Zink mit 10 %iger Salzsäure, Aceton und Diethylether nacheinander gewaschen und anschließend getrocknet.
Es wurden 0,40 g (6,12 mmol) aktiviertes Zink unter Argon in den Reaktionskolben eingewogen und mit 2 ml Tetrahydrofuran versetzt. Danach wurde unter Inertisierung mit Argon auf ca. 65 °C erwärmt. Innerhalb von 15 min wurde eine Lösung von 5,56 mmol des entsprechenden optisch aktiven Acyloxynitrils (Tabelle 2) in 1 ml THF zugetropft. Nach einer Rührzeit von 2 Stunden bei ca. 65 °C wurde abgekühlt und bei einer Temperatur von unter 0 °C 2 ml gesättigte Ammonchloridlösung zugetropft und mit 4 ml Wasser sowie 4 ml Tetrahydrofuran verdünnt. Anschließend wurde auf Raumtemperatur erwärmt und die Phasen getrennt. Von der organischen Phase wurde ein Teil des Lösungsmittels abdestilliert und 5 ml Salzsäure 1:1 zugesetzt. Danach wurde 24 Stunden lang bei 60-70 °C hydrolysiert.
Anschließend wurde das Reaktionsgemisch vom Lösungsmittel befreit und der Rückstand mit 20 ml destilliertem Wasser versetzt. Die wäßrige Phase wurde mit 2 M Natronlauge auf pH 9,5 gestellt. Nach Extraktion mit Ethylacetat und Phasentrennung wurde die wäßrige Phase mit 10 %iger Salzsäure auf pH 1 gestellt und dreimal mit Ethylacetat extrahiert. Die organischen Phasen wurden getrocknet und vom Lösungsmittel befreit. Die Enantiomerenreinheit wurde mittels GC an einer Cyclodextrinsäule bestimmt.

**Tabelle 2:**

| Acyloxynitril | ee | Hydroxyfuranon | Ausbeute | ee |
|---|---|---|---|---|
| (S)-2-(2-Brompropionyloxy)-phenylacetonitril | > 98 % | (5S)-4-Hydroxy-3-methyl-5-phenyl-2(5H)-furanon | 70 % | 93 % |
| | | | | |
| (S)-2-(2-Brompropionyloxy)-nonanonitril | 93 % | (5S)-4-Hydroxy-3-methyl-5-heptyl-2(5H)-furanon | 71 % | 91 % |
| | | | | |
| (S)-2-(2-Brompropionyloxy)-2-phenylpropionitril | 96 % | (5S)-4-Hydroxy-3,5-dimethyl-5-phenyl-2(5H)-furanon | 75 % | 96 % |

## Patentansprüche

1. Verfahren zur Herstellung von enantiomeren-angereicherten Aminofuranonen und Hydroxyfuranonen, **dadurch gekennzeichnet, dass**
a) ein enantiomeren-angereichertes Cyanhydrin mittels eines Acylierungsmittels acyliert, anschließend
b) bei 40 bis 90°C in Anwesenheit von Zink oder einer Zinkverbindung zu dem korrespondierenden enantiomeren-angereicherten Aminofuranon cyclisiert wird, welches
c) gegebenenfalls durch saure Hydrolyse in das entsprechende enantiomerenangereicherte Hydroxyfuranon überführt wird.

2. Verfahren nach Anspruch 1, **dadurch gekennzeichnet, dass** als Ausgangsverbindung ein optisch aktives Aldehyd- oder Keton-Cyanhydrin eingesetzt wird, das die (S)- oder (R)- Form in einem Anteil von über 95% enthält.

3. Verfahren nach Anspruch 1, **dadurch gekennzeichnet, dass** durch Acylierung des Cyanhydrins eine Verbindung der Formel 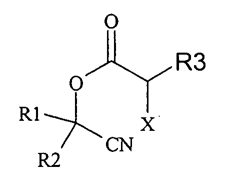 erhalten wird, in einer der beiden Reste R1 oder R2 H bedeuten kann; oder R1 und R2 gleich oder verschieden sind und eine lineare, verzweigte oder cyclische C₂-C₁₈-Alkylgruppe, C₂-C₁₈-Alkylengruppe oder C₂-C₁₈-Alkylidengruppe, die gegebenenfalls durch Halogen-, Ether-, Alkohol-, Acyl-, Carbonsäure-, Carbonsäureester-, Nitro- oder Azidogruppen oder durch gegebenenfalls substituierte Aryloder Heteroarylgruppen ein- oder mehrfach substituiert sein kann oder ein gegebenenfalls substituiertes Aralkyl, Aryl oder Heteroaryl bedeuten,
R3 einen aliphatischen, linearen oder verzweigten C₁ bis C₁₈-Alkylrest oder einen Arylrest mit 6 bis 20 C-Atomen und X ein Halogenid aus der Gruppe Fluor, Brom, Chlor und Jod bedeutet.

4. Verfahren nach Anspruch 1, **dadurch gekennzeichnet, dass** die Cyclisierung in Schritt b) in Gegenwart von Zink, das gegebenenfalls durch Jod oder Kupfer aktiviert werden kann, bei 40-90°C erfolgt, wodurch Verbindungen der Formel (IV) 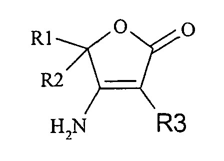 in der R1, R2 und R3 wie oben definiert sind, in Ausbeuten bis zu über 90% und in einer Enantiomerenreinheit von >90 % erhalten werden.

5. Verfahren nach Anspruch 1, **dadurch gekennzeichnet, dass** die in Schritt c) erhaltenen Aminofuranone durch saure Hydrolyse in die korrespondierenden Hydroxyfuranone überführt werden, wobei die Hydrolyse nach Isolierung des entsprechenden Aminofuranons oder direkt im Anschluss an die Cyclisierung des Schrittes b) erfolgen kann.
